# EUROPEAN PATENT APPLICATION

(11) **EP 4 541 346 A1**
(43) Date of publication of application: **23.04.2025**
(21) Application number: 23827457.5
(22) Date of filing: 16.06.2023
(51) Int. Cl.: A61K 8/9789, A61K 8/60, A61Q 19/00, A61Q 19/08, A61Q 19/02

(54) **HIGH-PURITY GINSENG EXOSOME COMPOSITION AND USE THEREOF IN FUNCTIONAL COSMETICS**

(30) Priority: 20.06.2022 KR 20220075171
(71) Applicant: Exostemtech Co., Ltd., Ansan-si, Gyeonggi-do 15588 (KR); Industry-University Cooperation Foundation Hanyang University ERICA Campus, Ansan-si, Gyeonggi-do 15588 (KR)
(72) Inventor: CHO, Yong Woo, Seongnam-si Gyeonggi-do 13530 (KR); CHOI, Ji Suk, Siheung-si, Gyeonggi-do 14977 (KR); CHOI, Young Chan, Chuncheon-si, Gangwon-do 24206 (KR); LEE, Hye Ri, Ansan-si, Gyeonggi-do 15497 (KR)
(74) Representative: Diehl & Partner
(86) International application number: PCT/KR2023/008414
(87) International publication number: WO 2023/249340

(57) **Abstract**

The present invention relates to a functional cosmetic composition comprising plant exosomes derived from ginseng as an active ingredient and a functional cosmetic containing the same. Ginseng exosomes have a bilipid layer structure and are rich in ginsenosides Rb1 and Rg1, which influence skin improvement and whitening. It has been proven that these powerful functional materials, ginsenosides Rb1 and Rg1, are absorbed in large quantities through ginseng exosomes into skin cells, enhance the synthesis of collagen and hyaluronic acid in skin cells, and have the efficacy of inhibiting melanin synthesis. Therefore, the ginseng exosomes rich in ginsenosides Rb1 and Rg1, presented in the present invention, can be beneficially used in functional cosmetic compositions for skin aging prevention, skin whitening, wrinkle improvement and skin cell regeneration.

## Description

### TECHNICAL FIELD

The present application claims the priority based on Korean Patent Application No. 10-2022-0075171 filed on June 20, 2022, and the entire contents disclosed in the description and drawings of the corresponding application are incorporated in the present application.

The present invention relates to a cosmetic composition containing ginseng exosomes as an active ingredient and a functional cosmetic comprising the same.

### BACKGROUND ART

Exosomes are reported that they are nano-sized microvesicles secreted from various cells and contain proteins, lipids, mRNA and miRNA, and act on the endocrine system and play a role of an important mediator of intercellular communication. Such a role is attracting attention as a new therapeutic agent which can overcome the engraftment rate and immunogenicity that are problems of conventional stem cell therapeutic agents.

According to recent studies, exosomes isolated from edible plants are known to have very high stability and biocompatibility in the body, as they do not have toxicity or immunogenicity compared to exosomes secreted from mammal cells. In addition, it was reported that exosomes secreted from mammal cells contain about 20% cholesterol, but plant exosomes have no cholesterol. In particular, compared with animal tissue or cell cultured solutions, raw plants have advantages in that they can be obtained in large quantities.

Various proteins are comprised inside exosomes depending on the plant of origin, but little is known about ginseng exosomes until now. In most studies, the characteristics of plant exosomes are identified by the size or shape of the exosomes, and a method for extracting only exosomes by selecting an appropriate size to extract plant exosomes is required.

In case of conventional technology, an attempt to extract plant exosomes through a density gradient ultracentrifugation method using a sucrose gradient has been made, but such a method is difficult to be used for a production process for commercialization of exosomes, and has problems that sucrose is mixed into the extract. In addition, with the density gradient ultracentrifugation method using a sucrose gradient, it is impossible to obtain a high-purity exosome composition in which exosomes are highly concentrated at a content of 10⁷ or more per unit volume of 1 mL of the extract. Furthermore, there is a method of filtering isolated extracellular vesicles to obtain a substance with a size smaller than pores of a filtration filter, and this is very disadvantageous for long-term exosome extraction, as when exosomes are extracted from a large amount of plant juice, impurities or exosomes are adsorbed and accumulated in the filter pores and the filtration efficiency is dramatically reduced.

Throughout the present description, numerous references are referred and citations thereof are indicated. The disclosures of the cited references are incorporated in the present description by reference in their entirety and the level of the technical field to which the present invention pertains and the contents of the present invention will be more clearly described.

### DISCLOSURE

### Technical Problem

An object of the present invention is to provide a high-purity ginseng exosome composition, comprising ginseng-derived exosomes as an active ingredient. More specifically, the present invention relates to the efficacy of collagen synthesis, hyaluronic acid synthesis and whitening of human skin tissue of the plant exosomes comprising ginsenosides Rg1 and Rb1 isolated·purified from ginseng, and an object of the present invention is to provide a cosmetic composition comprising ginseng exosomes having skin regeneration and whitening functions as an active ingredient.

Another object of the present invention is to provide a functional cosmetic for skin protection, wrinkle improvement, skin barrier improvement and skin whitening and the like, prepared by using the composition as a cosmetic raw material.

Other object of the present invention is to provide a method for skin whitening, comprising applying a cosmetic composition comprising ginseng exosomes to a subject.

Other object of the present invention is to provide a cosmetic use of a cosmetic composition comprising ginseng exosomes for skin whitening.

Other object of the present invention is to provide a cosmetic use of a cosmetic composition comprising ginseng exosomes for wrinkle improvement and skin regeneration, and skin barrier improvement.

More specifically, an object of the present invention is to provide the following embodiments.

Embodiment 1. A high-purity ginseng exosome composition, comprising ginseng-derived exosomes as an active ingredient.

Embodiment 2. A method for treating skin of a subject in need thereof, comprising applying ginseng exosomes isolated from ginseng or a ginseng exosome composition into a subject, wherein the isolated ginseng exosomes or ginseng exosome composition provides effects of anti-aging, anti-oxidation, collagen production, hyaluronic acid production, skin whitening, wrinkle improvement and/or skin cell regeneration on skin.

Embodiment 3. A use of ginseng exosomes isolated from ginseng or a ginseng exosome composition for providing (or producing) a cosmetic effect on skin (or human skin), wherein the isolated ginseng exosomes or ginseng exosome composition provides effects of anti-aging, anti-oxidation, collagen production, hyaluronic acid production, skin whitening, wrinkle improvement and/or skin cell regeneration on skin.

Embodiment 4. According to any one of the preceding embodiments, the ginseng exosomes isolated from ginseng are comprised at a content of 1 x 10⁷ to 1 x 10¹⁴ per unit volume of 1 mL of the composition.

Embodiment 5. According to any one of the preceding embodiments, the composition is a cosmetic composition.

Embodiment 6. According to any one of the preceding embodiments, the composition has at least one skin cosmetic use selected from the group consisting of anti-aging, anti-oxidation, collagen production, hyaluronic acid production, skin whitening, wrinkle improvement and/or skin cell regeneration.

Embodiment 7. According to any one of the preceding embodiments, the composition is a formulation selected from the group consisting of aqueous solution, suspension, emulsion, cream, frozen solution, spray-dried powder, and freeze-dried powder.

Embodiment 8. According to any one of the preceding embodiments, the ginseng exosomes isolated from ginseng comprise ginsenoside Rb1 and ginsenoside Rg1.

Embodiment 9. According to any one of the preceding embodiments, a cosmetic comprising ginseng exosomes isolated from ginseng or a ginseng exosome composition.

Embodiment 10. According to any one of the preceding embodiments, the cosmetic is a formulation selected from the group consisting of mist, serum, nutritional cosmetic water, soft cosmetic water, soft water, emulsion, skin lotion, skin softener, skin toner, astringent, lotion, milk lotion, moisture lotion, nutritional lotion, massage cream, nutritional cream, moisture cream, hand cream, foundation, powder, makeup base, essence, nutritional essence, pack, soap, cleansing foam, cleansing lotion, cleansing cream, body lotion, body cleanser, facial cleanser, treatment, cosmetic solution, cosmetic pack, ointment, gel, liniment, liquid, patch, spray, bath preparation, sunscreen agent, sun oil, and hair product.

Embodiment 11. According to any one of the preceding embodiments, the cosmetic is a functional cosmetic having at least one efficacy selected from the group consisting of skin aging prevention, skin whitening, wrinkle improvement and skin cell regeneration.

Embodiment 12. According to any one of the preceding embodiments, the cosmetic comprises the ginseng exosomes at a content of 1 x 10⁶ to 1 x 10¹² per unit volume of 1 mL.

Embodiment 13. A method for preparing the ginseng exosome composition according to any one of the preceding embodiments, which is a method comprising performing centrifugation; and performing tangential-flow filtration (TFF), wherein a high-purity ginseng exosome composition comprising ginseng-derived exosomes at a content of 1 x 10⁷ to 1 x 10¹⁴ per unit volume of 1 mL of the composition is obtained by the method.

Other objects and advantages of the present invention will become more apparent from the following detailed description of the invention, claims and drawings.

### Technical Solution

One aspect of the present invention is to provide a high-purity ginseng exosome composition, comprising ginseng-derived exosomes as an active ingredient. The ginseng-derived exosomes may be comprised at a content of 1 X 10⁶ to 1 X 10¹³, preferably, 1 X 10⁷ to 1 x 10¹⁴, more preferably, 1 x 10¹¹ to 1 × 10¹⁴, much more preferably, 1 x 10¹² to 1 x 10¹⁴ per unit volume of 1 mL of the composition.

The term used in the present invention, "exosome" collectively refers to exosomes having a size of about 50 ~ 300 nm, preferably, a size of 50 ~ 200 nm, extracted from ginseng, or extracellular vesicles including exosomes. Ginseng exosomes form particles with a uniform size of 50 to 200 nm in the form made of bilipid membranes.

It has been known that conventional various plant extracts comprise anti-aging and anti-oxidant substances, but there were problems that it was difficult to apply them to cosmetic raw materials because it was difficult to pass through the skin barrier in the form of a water-soluble extract, and also, the active ingredient was exposed to an extraction solvent during an extraction process and was easily destroyed. On the other hand, exosomes are nanoparticles made of bilipid membranes and are absorbed into skin cells by receptor-mediated endocytosis and membrane fusion, and in addition, active ingredients comprised inside are not directly exposed, so they are not easily degenerated and biological activity can be maintained for a long period of time, so they are very advantageous to industrially use them in the cosmetic industry.

Ginseng, which is the raw material plant of the present invention, belongs to *Panax ginseng,* which is one kind of the genus of Ginseng. As a raw material plant part, the whole ginseng or at least one part including flowers, stems, buds and fruits and the like may be mixed and used.

In a preferable embodiment, the ginseng exosome composition of the present invention is a cosmetic composition, and the composition may have at least one skin cosmetic use selected from the group consisting of anti-aging, anti-oxidation, collagen production, hyaluronic acid production, skin whitening, wrinkle improvement and skin cell regeneration.

The ginseng exosomes suggested in the present invention comprise an excessive amount of ginsenosides Rg1 and Rb1, and have functions of improving collagen production and hyaluronic acid production of human skin and inhibiting melanin synthesis.

In the present invention, the ginseng exosomes have skin regeneration and whitening functions by functional substances including ginsenosides derived from ginseng, which is a raw material plant. Specifically, the ginseng exosomes containing ginsenoside Rg1 and ginsenoside Rb1, polyphenol, and the like known to have anti-oxidant function in large quantities exhibit efficacy of skin regeneration and whitening and the like by inhibiting active oxygen accumulated in skin cells.

Ginseng exosomes have anti-aging and anti-oxidation functions by polyphenol components comprised by themselves, and they promote degradation of active oxygen present outside the cells and active oxygen accumulated inside the cells.

In particular, the ginseng exosomes having the anti-oxidation function are absorbed into skin cells by receptor-mediated endocytosis and membrane fusion, and the absorbed ginseng exosomes stimulate anti-oxidant signaling mechanisms inside skin cells to increase expression of genes such as SOD, catalases (CAT) and the like and exhibit anti-aging and anti-oxidation efficacy.

The ginseng exosomes developed in the present invention contain natural steroid glycosides or ginsenosides, which are one kind of triterpene saponin in large quantities. It has been demonstrated that among them, ginsenosides Rg1 and Rb1 have anti-oxidant action mechanisms, and have not only skin improvement such as skin whitening and collagen regeneration and the like, but also clinical efficacy such as immunity enhancement, fatigue improvement, blood flow improvement, memory improvement and the like.

In one embodiment, the skin regeneration and whitening cosmetic composition comprising ginseng exosomes of the present invention as an active ingredient may be provided as a form selected from the group consisting of aqueous solution, suspension, emulsion, cream, frozen solution, spray-dried powder and freeze-dried powder.

Another aspect of the present invention is to provide a cosmetic comprising the skin regeneration and whitening cosmetic composition comprising ginseng exosomes as an active ingredient.

The cosmetic may apply and mix general components used in preparation of general skin cosmetics, for example, oil, water, surfactants, moisturizers, lower alcohols, thickeners, chelating agents, pigments, preservatives, flavorings and the like in a required amount.

In addition, the cosmetic may be prepared in a formulation selected from the group consisting of mist, serum, nutritional cosmetic water, soft cosmetic water, soft water, emulsion, skin lotion, skin softener, skin toner, astringent, lotion, milk lotion, moisture lotion, nutritional lotion, massage cream, nutritional cream, moisture cream, hand cream, foundation, powder, makeup base, essence, nutritional essence, pack, soap, cleansing foam, cleansing lotion, cleansing cream, body lotion, body cleanser, facial cleanser, treatment, cosmetic solution, cosmetic pack, ointment, gel, liniment, liquid, patch, spray, bath preparation, sunscreen agent, sun oil, and hair product, and various kinds of common carriers and additives which are suitable for each formulation thereof and are known in the art may be comprised.

Such a cosmetic of the present invention may be a functional cosmetic having at least one efficacy selected from the group consisting of collagen production, hyaluronic acid production, skin whitening, wrinkle improvement, skin barrier improvement and skin cell regeneration.

In the present invention, the ginseng exosomes extracted from ginseng may be comprised at a content of the number of particles of 1 X 10 or more, 1 X 10² or more, 1 X 10³ or more, 1 X 10⁴ or more, 1 X 10⁵ or more or 1 X 10⁶ or more per unit volume of 1 mL of the cosmetic, and preferably, they may be comprised at a content of particles of 1 X 10⁶ to 1 X 10¹², more preferably, 1 X 10⁶ to 1 X 10¹², much more preferably, 1 X 10⁷ to 1 X 10¹².

Other aspect of the present invention is to provide a method for preparation of a high-purity ginseng composition comprising the ginseng exosomes as an active ingredient, which is a method for preparation comprising performing centrifugation; and performing tangential-flow filtration (TFF).

More specifically, the method for preparation of the present invention may comprise crushing ginseng; performing microfiltration and centrifugation; and performing tangential-flow filtration (TFF).

The process of crushing raw material plants may include a process of mechanically crushing a mixture of the raw material plants and buffer solution mixed within a range of 1:1 to 1:10 as a weight ratio, and this may be performed by mechanically crushing the plant mixture with a rotating blade at a rate of 10 to 1,000 rpm.

The microfiltration method may be performed by filtering the plant mixture with a filtration membrane having pores with a size of 10 µm to 200 nm, and preferably, it may be filtered from at least one combination of filtration membranes having pores different from each other.

The centrifugation method may be selected from low-speed centrifugation, high-speed centrifugation, ultracentrifugation and combinations thereof, and preferably, it may include ultracentrifugation. The low-speed centrifugation can achieve a speed of 6,000 rpm (6,000 xg) or less, and be mainly used for centrifugation of samples that are easily precipitated such as cells or nuclei or the like, and the high-speed centrifugation has a maximum speed of about 20,000-25,000 rpm (60,000 xg), and the ultracentrifugation refers to a centrifugation method with a maximum speed of about 40,000-80,000 rpm (600,000 xg).

The tangential-flow filtration (TFF) is a filtration method in which a solution flows in a direction perpendicular to a filtration membrane, and impurities with a small size present in the solution are filtered, and exosomes with a large size are isolated. It can minimize adsorption of exosomes to pores of a filtration filter or clogging of membrane pores compared to the conventional filtration method, and it is easy to apply to process scale-up and cGMP (cosmetic good manufacturing procedure) processes. The tangential-flow filtration may be at least one selected from the group consisting of hollow fiber TFF and membrane TFF which can perform ultrafiltration, and preferably, it may use a TFF filter having a molecular weight cutoff (MWCO) of 100,000 Da to 500,000 Da.

As such, the ginseng exosomes according to the present invention may be isolated from ginseng through mechanical crushing, stepwise ultracentrifugation and tangential flow filtration (TFF) methods, and these exosomes contain a large amount of polyphenols and ginsenosides and the like which have skin regeneration, whitening and anti-oxidation functions.

### Advantageous Effects

Ginseng exosomes have a bilipid membrane structure and are absorbed into skin cells, and ginsenosides Rg1 and Rb1, polyphenol, and the like comprised within the absorbed ginseng exosomes exhibit anti-oxidant efficacy by stimulating anti-oxidant signaling mechanisms in skin cells to increase expression of various anti-oxidant genes. They have various functional cosmetic uses such as collagen production, hyaluronic acid production, whitening and anti-aging, skin cell activation, and the like, derived from the anti-oxidant efficacy of the ginsenosides Rg1 and Rb1, and polyphenol contained in the ginseng exosomes. In addition, the excellent stability of the ginseng exosomes can be used for functional cosmetics in various forms such as aqueous solutions, frozen solutions and freeze-dried powders, and the like.

### DESCRIPTION OF DRAWINGS

FIG. 1 is a process schematic diagram of a plant exosome extraction method for isolating plant exosomes from plant raw materials with high purity and high yield.
FIG. 2a is the results of analyzing the characteristics of aloe exosomes by nanoparticle tracking analysis (NTA).
FIG. 2b is the results of analyzing the characteristics of garlic exosomes by NTA.
FIG. 2c is the results of analyzing the characteristics of sea mustard exosomes by NTA.
FIG. 2d shows the results of confirming the number of ginseng exosomes (left) and the results of measuring the protein content in the exosomes (right) by NTA.
FIG. 3 shows the results of evaluating the total polyphenol content in the ginseng exosomes.
FIG. 4 shows the results of evaluating morphological stability of the ginseng exosomes using cryo-TEM after storing them at frozen (-70 °C) and room temperature (25 °C) for 3 months.
FIG. 5a shows the ginsenoside Rb1 detection reference.
FIG. 5b shows the ginsenoside Rg1 detection reference.
FIG. 5c shows the results of detecting ginsenoside Rg1 and ginsenoside Rb1 in the ginseng exosomes.
FIG. 5d shows the results of detecting the total polyphenol compounds in the ginseng exosomes.
FIG. 6 shows the results of confirming the efficacy of the ginseng exosomes to increase intracellular collagen production.
FIG. 7 shows the results of confirming the efficacy of the ginseng exosomes to increase intracellular hyaluronic acid production.
FIG. 8 shows the results of confirming the efficacy of the ginseng exosomes to inhibit extracellular and intracellular melanin formation.

### BEST MODE

Hereinafter, the present invention will be described in more detail by examples. These examples are intended to describe the present invention more specifically only, and it will be obvious to those skilled in the art to which the present invention pertains that the scope of the present invention is not limited by these examples.

### Example

### <Example 1> Isolation of exosomes from various plant raw materials

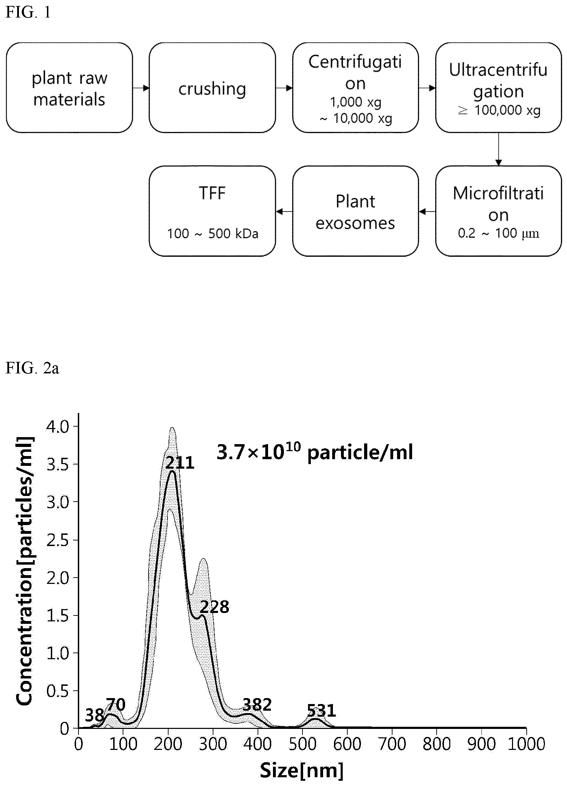

By performing ultracentrifugation and tangential-flow filtration (TFF), plant exosomes can be isolated from various plant raw materials with high-purity and high yield (FIG. 1). In the present example, using aloe, garlic, sea mustard and ginseng, high-purity plant exosome compositions were prepared.

### Preparation of an aloe-derived exosome composition

Aloe exosomes were isolated from a raw material plant, aloe, by using methods of ultracentrifugation and tangential-flow filtration (TFF). Specifically, aloe peel was placed in a blender with phosphate-buffered saline (PBS) at a weight ratio of 1:2(w/w) and then crushed. By centrifuging at 1,000 Xg for 10 minutes, the supernatant was collected, and the supernatant was sequentially centrifuged at 2000 Xg for 20 minutes, at 3,000 Xg for 30 minutes, and at 10,000 Xg for 60 minutes. After that, the supernatant was ultracentrifuged at 4°C at 100,000 Xg for 70 minutes, and through this process, vacuoles were removed. Finally, the supernatant was discarded and the remaining pellets were suspended and then exosomes were isolated by the TFF system. Specifically, they were filtered using a filter having a molecular cut off of 100 to 500 kDa in the TFF system, and other impurity particles smaller than the pores of the filter were removed and the solution containing the exosomes was concentrated.

As a result of performing ultrafiltration, TFF as such to extract aloe peel-derived exosomes, the extracted aloe peel-derived exosomes were confirmed to have a spherical microstructure of less than 200 nm, and through nanoparticle tracking analysis (NTA), it was confirmed that a high-purity exosome composition, in which the aloe exosomes were concentrated at a concentration of 1 x 10^{9~11} per unit volume of 1 mL, was obtained (FIG. 2a).

### (2) Preparation of a garlic-derived exosome composition

Garlic-derived exosomes were isolated from a raw material plant, garlic, by using methods of ultracentrifugation and tangential-flow filtration (TFF). Specifically, garlic of which peel was removed was placed in a blender with phosphate-buffered saline (PBS) at a weight ratio of 1:2(w/w) and then crushed. By centrifuging at 1,000 Xg for 10 minutes, the supernatant was collected, and the supernatant was sequentially centrifuged at 2000 Xg for 20 minutes, at 3,000 Xg for 30 minutes, and at 10,000 Xg for 60 minutes. After that, the supernatant was ultracentrifuged at 4°C at 100,000 Xg for 70 minutes, and through this process, vacuoles were removed. Finally, the supernatant was discarded and the remaining pellets were suspended and then exosomes were isolated by the TFF system. Specifically, they were filtered using a filter having a molecular cut off of 100 to 500 kDa in the TFF system, and other impurity particles smaller than the pores of the filter were removed and the solution containing the garlic-derived exosomes was concentrated.

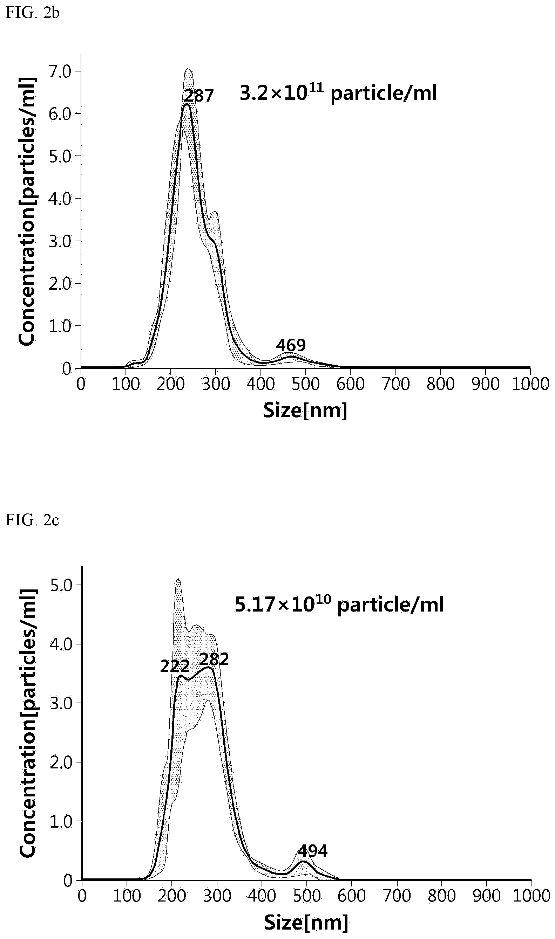

As a result of performing ultrafiltration, TFF as such to extract garlic-derived exosomes, the garlic-derived exosomes were confirmed to have a spherical microstructure of less than 200 nm, and through nanoparticle tracking analysis (NTA), it was confirmed that a high-purity exosome composition, in which the garlic exosomes were concentrated at a concentration of 1 x 10^{9~11} per unit volume of 1 mL, was obtained (FIG. 2b).

### (3) Preparation of a sea mustard-derived exosome composition

Sea mustard-derived exosomes were isolated from a raw material plant, sea mustard, by using methods of ultracentrifugation and tangential-flow filtration (TFF). Specifically, moisture-removed sea mustard was removed was placed in a blender with phosphate-buffered saline (PBS) at a weight ratio of 1:2(w/w) and then crushed. By centrifuging at 1,000 Xg for 10 minutes, the supernatant was collected, and the supernatant was sequentially centrifuged at 2000 Xg for 20 minutes, at 3,000 Xg for 30 minutes, and at 10,000 Xg for 60 minutes. After that, the supernatant was ultracentrifuged at 4°C at 100,000 Xg for 70 minutes, and through this process, vacuoles were removed. Finally, the supernatant was discarded and the remaining pellets were suspended and then exosomes were isolated by the TFF system. Specifically, they were filtered using a filter having a molecular cut off of 100 to 500 kDa in the TFF system, and other impurity particles smaller than the pores of the filter were removed and the solution containing the exosomes was concentrated.

As a result of performing ultrafiltration, TFF as such to extract sea mustard-derived exosomes, the sea mustard-derived exosomes were confirmed to have a spherical microstructure of less than 200 nm, and through nanoparticle tracking analysis (NTA), it was confirmed that a high-purity exosome composition, in which the sea mustard exosomes were concentrated at a concentration of 1 x 10^{9~11} per unit volume of 1 mL, was obtained (FIG. 2c).

### (4) Preparation of a ginseng-derived exosome composition

Ginseng exosomes were isolated from a raw material plant, ginseng, by using methods of ultracentrifugation and tangential-flow filtration (TFF). Specifically, ginseng was removed was placed in a blender with phosphate-buffered saline (PBS) at a weight ratio of 1:2(w/w) and then crushed. By centrifuging at 1,000 Xg for 10 minutes, the supernatant was collected, and the supernatant was sequentially centrifuged at 2000 Xg for 20 minutes, at 3,000 Xg for 30 minutes, and at 10,000 Xg for 60 minutes. After that, the supernatant was ultracentrifuged at 4°C at 100,000 Xg for 70 minutes, and through this process, vacuoles were removed. Finally, the supernatant was discarded and the remaining pellets were suspended and then exosomes were isolated by the TFF system. Specifically, they were filtered using a filter having a molecular cut off of 100 to 500 kDa in the TFF system, and other impurity particles smaller than the pores of the filter were removed and the solution containing the exosomes was concentrated.

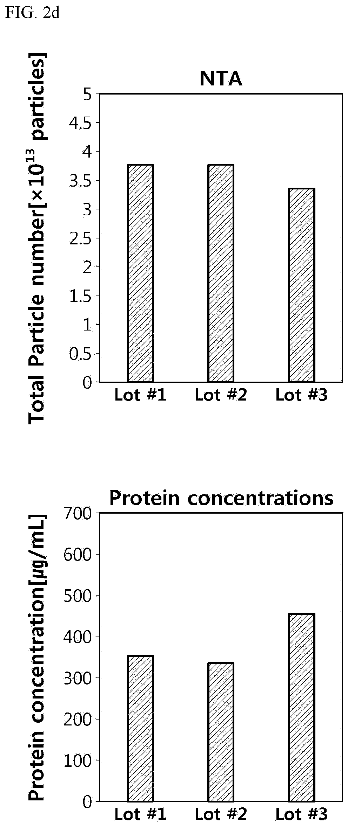

As a result of performing ultrafiltration, TFF as such to extract ginseng-derived exosomes, the ginseng-derived exosomes were confirmed to have a spherical microstructure of 50 to 300 nm, and through nanoparticle tracking analysis (NTA), it was confirmed that a high-purity exosome composition, in which the ginseng exosomes were concentrated at a concentration of 1 x 10¹³ or more per unit volume of 1 mL, was obtained (left graph of FIG. 2d).

When the combination of ultracentrifugation and TFF methods was applied to ginseng particularly among various plant raw materials as above, it was confirmed that a high-purity exosome composition with significantly high particle counts per unit volume could be achieved. Thus, additional studies were performed using ginseng exosomes confirmed to provide the exosome composition with the highest purity below.

### <Example 2> Evaluation of characteristic of ginseng exosomes

For the ginseng exosomes isolated in Example 1 above, evaluation of characteristics of exosomes was performed by the following method. The size of the microparticles was analyzed by nanoparticle tracking analysis (NTA), and as described above, the ginseng exosomes were confirmed to have a size of 50 to 300 nm, and by nanoparticle tracking analysis (NTA), it was confirmed that the ginseng exosomes produced once had a yield of about 1 x 10¹³ or more per lot (left graph of FIG. 2d). In addition, the protein content in the exosomes was measured using Micro BCA^{™} Protein Assay Kit of Thermo Scientific company, and it was confirmed that proteins of 10 to 1,000 µg/mL per unit volume of 1 mL were contained (right graph of FIG. 2d).

### <Example 3> Evaluation of total polyphenol content of ginseng exosomes

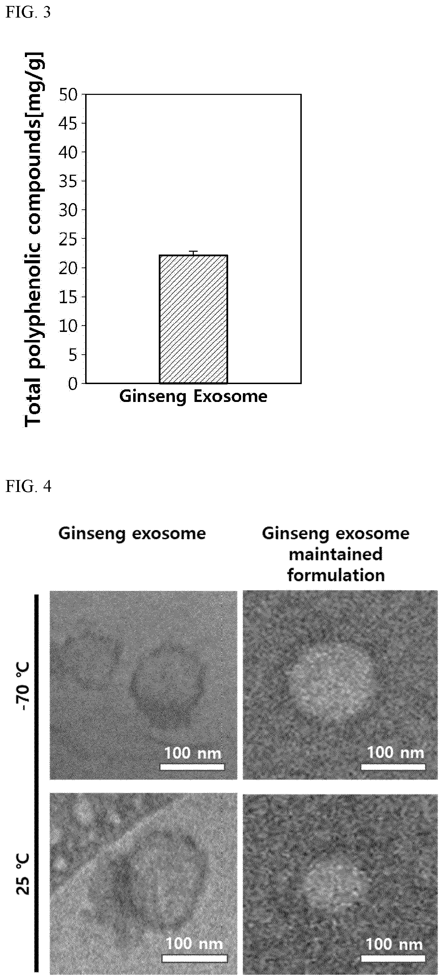

For the ginseng exosomes isolated in Example 1 above, the total polyphenol content in the exosomes was evaluated by the following method (FIG. 3). The ginseng exosomes isolated in Example 1 was extracted with an ultrasonic shaker by 1 mg/mL in water and the ginseng exosomes were used as specimens. The ginseng exosomes of 20 mg were ultrasonically extracted with 5 mL purified water at a room temperature for 10 minutes and were filtered with a 0.45um PVDF membrane filter and used as a test solution. The test solution of 1 mL and purified water of 7.5 mL were placed in a test tube and 2N-Folin-ciocalteu Reagent 0.5 mL each was added to the test tube, and 23% Sodium carbonate solution 1 mL each was added, and they were reacted in a dark place at a room temperature for 10 minutes. As a standard material, Gallic acid 1 mg was ultrasonically extracted with 1 mL purified water at a room temperature for 10 minutes, and were filtered with a 0.45um PVDF membrane filter and used as a standard undiluted solution (250, 125, 62.5, 31.25, 15.625, 7.8125, 3.90625 ppm), and by concentration, the standard solution was added to the test tube by 1 mL each, and 2 N-Folin-ciocalteu Reagent 0.5 mL each was added to the test tube, and 23% Sodium carbonate solution 1 mL each was added and they were reacted in a dark place at a room temperature for 1 hour. The absorbance was measured under the condition of 760 nm wavelength using Epoch Microplate Spectrophotometer, and the total polyphenol content in the ginseng exosomes was quantified. As the experimental result, it was confirmed that the total polyphenol content in the ginseng exosome composition was 20 to 30 mg/g. On the other hand, the total polyphenol content in the aloe exosome composition was confirmed to be 38 to 41 mg/g.

### <Example 4> Evaluation of stability of shape and storage of ginseng exosomes

The exosome shape stability was evaluated under the conditions of ultra freezing and room temperature storage of the ginseng exosomes isolated in Example 1 above. The ginseng exosomes were diluted at a concentration of 10⁸ p/mL to 10¹⁰ p/mL, and then a cosmetic raw material formation, in which trehalose and 1,3 propanediol, 1,2 hexanediol were added, was prepared. After preparation, it was stored at frozen (-70 °C) and room temperature (25 °C) for 3 months, and then the shape stability was evaluated using cryo-TEM. It was confirmed that both the ginseng exosomes and raw material formulation maintained the shape of the exosomes even after storage for 3 months or more (FIG. 4).

### <Example 5> Evaluation of ginsenoside content of ginseng exosomes

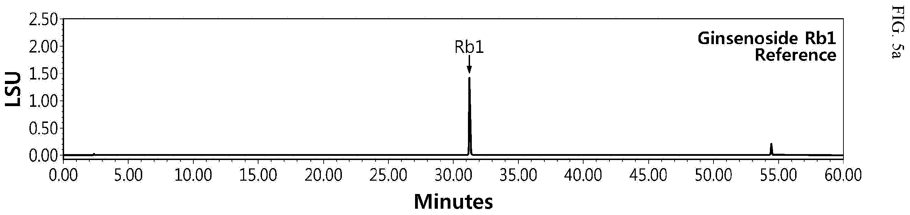

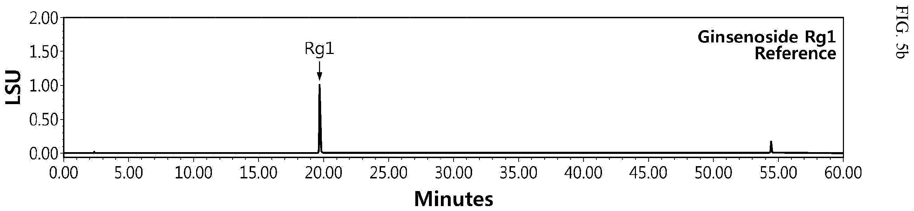

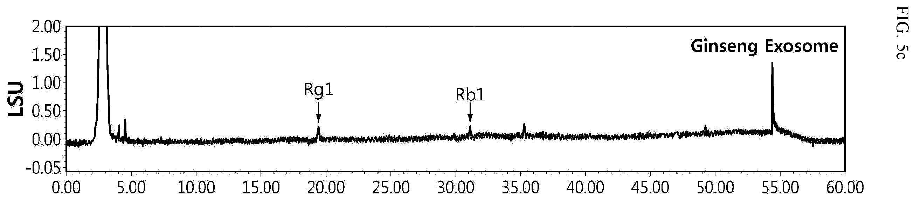

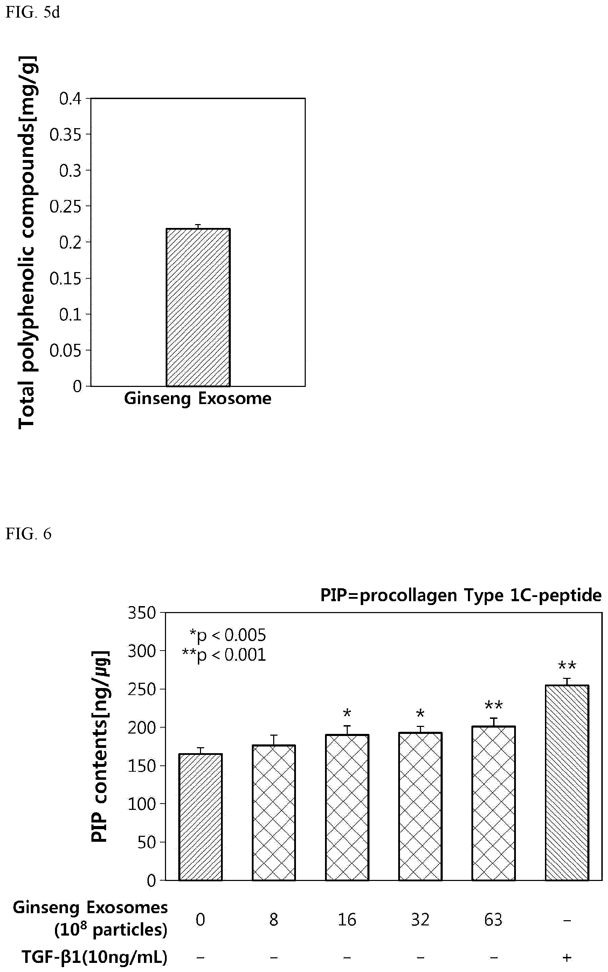

For the ginseng exosomes isolated in Example 1 above, the ginsenoside content in the exosomes was evaluated by the following method. The ginseng exosomes isolated in Example 1 were lyophilized and specimens were prepared. The freeze-dried exosomes were dissolved in methanol at a concentration of 117.9 mg/mL, and then melted with an ultrasonic shaker for 20 minutes, and then filtered with a 0.45um PVDF membrane filter, and used as a test solution. Ginsenosides Rg1 and Rb1 of 1mg each were dissolved in methanol and melted so as to be 1 mg/mL and then diluted (312.5, 195.313, 76.294, 47.684, 29.803 ppm), and used as a standard undiluted solution, and melted with an ultrasonic shaker for 20 minutes, and then filtered with a 0.45um PVDF membrane filter, and used as a standard solution. The contents of the ginsenoside Rg1 and ginsenoside Rb1 in the ginseng exosomes were measured using HPLC, and the mobile phase was purified under gradient solution conditions of purified water and acetonitrile. Ginsenoside Rg1 and ginsenoside Rb1 were detected in the ginseng exosomes, and it was confirmed that ginsenoside Rg1 of 0.378 to 0.386 mg, and ginsenoside Rb1 of 0.214 to 0.224 mg were contained inside the freeze-dried ginseng exosomes of 1 g (FIG. 5).

### <Example 6> Evaluation of in vitro collagen production efficacy of ginseng exosomes

In order to confirm the evaluation of cosmetic efficacy of the ginseng exosomes isolated in Example 1, the exosomes were treated to Human Dermal Fibroblasts (HDFs) to confirm the intracellular collagen production (PIP). Through cell viability analysis before measuring the collagen production, it was confirmed that there was no cytotoxicity at a concentration of 6.3 × 10⁸ p/mL or less. When measuring the intracellular collagen production, a cell culture medium having no a test substance was used as a solvent control, and as a positive control, TGF-β110 ng/mL was used. For confirmation of the collagen production, the sample comprising the ginseng exosomes was treated to HDFs cells of 5×10⁴, and after culturing for 24 hours, each cultured supernatant was centrifuged at 10,000rpm for 10 minutes, and with the supernatant in which debris was removed, for the amount of procollagen, the absorbance was measured at 450nm using Procollagen Type I C-Peptide (PIP) Kit.

In the ginseng exosomes, it was confirmed that the intracellular collagen production was increased at a concentration of 1.6×10⁸ to 6.3×10⁸ p/mL (FIG. 6). Preferably, the intracellular collagen production was increased at a concentration of 2×10⁸ to 6×10⁸ p/mL, and they have similar efficacy to the positive control, TGF-β1 (10ng/mL). In the present example, the ginseng exosome efficacy is not limited to collagen production efficacy, and includes all the functional cosmetic efficacy such as anti-oxidation, whitening, aging prevention, collagen production, and the like.

### <Example 7> Evaluation of in vitro hyaluronic acid production efficacy of ginseng exosomes

In order to confirm the cosmetic efficacy evaluation of the ginseng exosomes isolated in Example 1 above, the exosomes were treated to Human dermal fibroblasts (HDFs), which were primary cells isolated from adult skin to measure the intracellular hyaluronic acid production. Before measuring the hyaluronic acid production, through cell viability analysis, it was confirmed that there was no cytotoxicity at a concentration of 6.3×10⁸ p/mL or less. When measuring the intracellular hyaluronic acid production, a cell culture medium having no a test substance was used as a solvent control, and as a positive control, Retinoic acid 3 µg/mL was used. In the measurement of the intracellular hyaluronic acid production, the HDFs were inoculated in a 24-well plate by 5×10⁴ cells/well and were incubated for 24 hours, and then it was replaced with an FBS-free medium comprising the ginseng exosomes diluted by concentration and then they were incubated for 24 hours. After 24 hours, each culture supernatant was centrifuged at 10,000 rpm for 10 minutes, and with the supernatant in which debris was removed, for the hyaluronic acid production comprised in the medium, the absorbance was measured at 450nm using Human Hyaluronic Acid (HA) ELISA Kit.

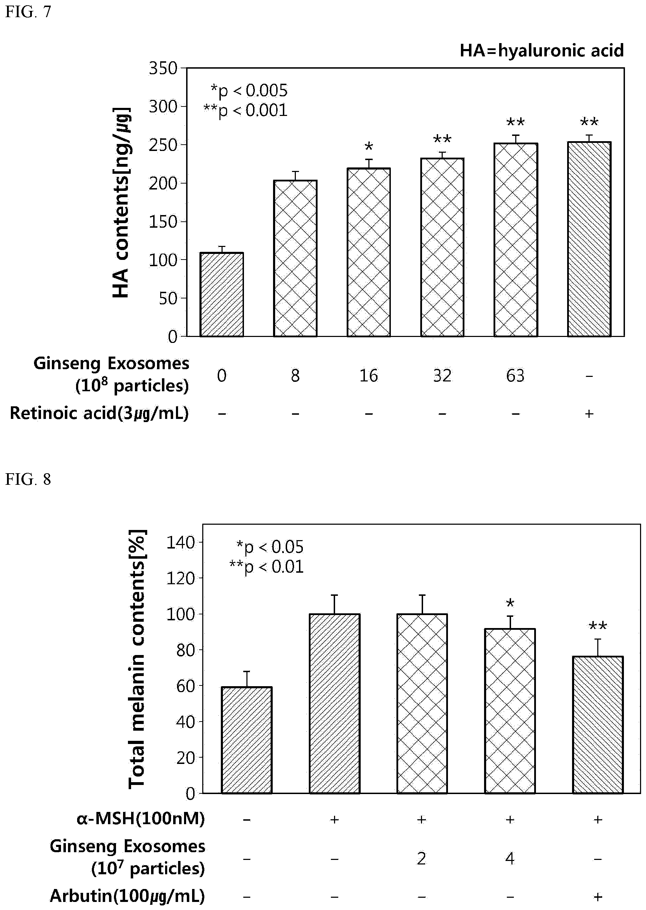

It was confirmed that the ginseng exosomes increased the intracellular hyaluronic acid production at a concentration of 1.6×10⁸ to 6.3×10⁸ p/mL (FIG. 7). Preferably, they increased the intracellular hyaluronic acid production at a concentration of 2×10⁸ to 4×10⁸ p/mL effectively, and they have similar efficacy to the positive control, retinoic acid. In the present example, the ginseng exosome efficacy is not limited to the hyaluronic acid production efficacy, and includes all the functional cosmetic efficacy such as anti-oxidation, whitening, aging prevention, collagen production, and the like.

### <Example 8> Evaluation of in vitro melanin production inhibitory efficacy of ginseng exosomes

In order to confirm the cosmetic efficacy evaluation of the ginseng exosomes isolated in Example 1 above, the exosomes were treated to skin melanoma cells (B16F10) to confirm whether they inhibited melanin production. Before measuring the melanin production, it was confirmed that there was no cytotoxicity at a concentration of 4×10⁷ p/mL or less. When measuring the melanin production, as a solvent control, a cell culture medium containing no test substance was used, and as a positive control, 100 mg/mL Arbutin was used. For the measurement of the total melanin production of the cells, the B16F10 cells were inoculated in a 6-well plate at a concentration of 1×10⁵ cells/well and incubated for 24 hours. After 24 hours, the medium of each well was removed, and the cells were washed with PBS, and then a new culture medium, in which the ginseng exosomes of 4×10⁷ p/mL and 100 nM α-MSH were contained and phenol red was not comprised, was added, and they were incubated for 72 hours. The incubated cells were used for measurement of the intracellular melanin amount, and the culture medium was used for measurement of the extracellular melanin amount. The total melanin production of the cells was calculated by summing each of the intracellular and extracellular melanin amounts. For measuring the intracellular melanin amount, the cultured B16F10 cells were washed with PBS and 1M NaOH was added to each well and they were dissolved at 60°C. For the cell lysate, the absorbance was measured at 400 nm, and then the melanin amount was calculated from a calibration curve using synthetic melanin, and the melanin amount was converted into the melanin amount per protein. For measurement of the extracellular melanin amount, the cell culture medium was centrifuged, and then the supernatant was transferred to a 96-well plate and the absorbance was measured at 400 nm. The melanin amount was calculated from a calibration curve using synthetic melanin, and the melanin amount was converted into the melanin amount per protein.

It was confirmed that the ginseng exosomes had an effect of inhibiting extracellular and intracellular melanin formation at a concentration of 4×10⁷ p/mL (FIG. 8). It was confirmed that the ginseng exosomes effectively inhibited extracellular and intracellular melanin formation. In the present example, the ginseng exosome efficacy is not limited to whitening efficacy, and includes all the functional cosmetic efficacy such as whitening, aging prevention, collagen production, and the like related to anti-oxidation.

## Claims

1. A high-purity ginseng exosome composition, comprising ginseng-derived exosomes as an active ingredient.

2. The ginseng exosome composition according to claim 1, wherein the ginseng-derived exosomes are comprised at a content of 1 x 10⁷ to 1 x 10¹⁴ per unit volume of 1 mL of the composition.

3. The ginseng exosome composition according to claim 1, wherein the composition is a cosmetic composition.

4. The ginseng exosome composition according to claim 1, wherein the composition has at least one skin cosmetic use selected from the group consisting of anti-aging, anti-oxidation, collagen production, hyaluronic acid production, skin whitening, wrinkle improvement and skin cell regeneration.

5. The ginseng exosome composition according to claim 1, wherein the composition is a formulation selected from the group consisting of aqueous solution, suspension, emulsion, cream, frozen solution, spray-dried powder, and freeze-dried powder.

6. The ginseng exosome composition according to claim 1, wherein the ginseng-derived exosomes comprise ginsenoside Rb1 and ginsenoside Rg1.

7. A cosmetic comprising the ginseng exosome composition according to any one claim of claim 1 to claim 6.

8. The cosmetic according to claim 7, wherein the cosmetic is a formulation selected from the group consisting of mist, serum, nutritional cosmetic water, soft cosmetic water, soft water, emulsion, skin lotion, skin softener, skin toner, astringent, lotion, milk lotion, moisture lotion, nutritional lotion, massage cream, nutritional cream, moisture cream, hand cream, foundation, powder, makeup base, essence, nutritional essence, pack, soap, cleansing foam, cleansing lotion, cleansing cream, body lotion, body cleanser, facial cleanser, treatment, cosmetic solution, cosmetic pack, ointment, gel, liniment, liquid, patch, spray, bath preparation, sunscreen agent, sun oil, and hair product.

9. The cosmetic according to claim 7, wherein the cosmetic is a functional cosmetic having at least one efficacy selected from the group consisting of skin aging prevention, skin whitening, wrinkle improvement and skin cell regeneration.

10. The cosmetic according to claim 7, wherein the cosmetic comprises the ginseng-derived exosomes at a content of 1 x 10⁶ to 1 x 10¹² per unit volume of 1 mL.

11. A method for preparing the ginseng exosome composition according to any one claim of claim 1 to claim 6, comprising
performing centrifugation; and
performing tangential-flow filtration (TFF),
wherein a high-purity ginseng exosome composition comprising ginseng-derived exosomes at a content of 1 x 10⁷ to 1 x 10¹⁴ per unit volume of 1 mL of the composition is obtained by the method.
